# EUROPEAN PATENT APPLICATION

(11) **EP 3 143 951 A1**
(43) Date of publication of application: **22.03.2017**
(21) Application number: 16188960.5
(22) Date of filing: 15.09.2016
(51) Int. Cl.: A61B 17/22

(54) **DEVICE FOR IMPROVING PASSAGE OF ELECTROHYDRAULIC LITHOTRIPSY PROBE**

(30) Priority: 15.09.2015 US 201562218788 P
(71) Applicant: Cook Medical Technologies LLC, Bloomington, IN 47404 (US)
(72) Inventor: WILLIAMS, Michael Lee, Pinnacle, NC 27043 (US); KENNEDY, Kenneth, Clemmons, NC 27102 (US)
(74) Representative: Garratt, Peter Douglas

(57) **Abstract**

A medical device is provided with a probe including an elongated shaft having a distal end, a proximal end, and an external wall. The probe also includes at least two conductors extending substantially along a longitudinal length of the probe. The probe also includes a strengthening element attached to an outer surface of the external wall of the elongated shaft. The probe is configured to slidably move within a working channel of a delivery device and the strengthening element is configured to limit buckling of the probe as a distal portion of the probe is advanced past a deflection point of the delivery device.

## Description

### CROSS-REFERENCE TO RELATED APPLICATIONS

The present patent document claims the benefit of the filing date under 35 U.S.C. § 119(e) of Provisional U.S. Patent Application Serial No. 62/218,788 filed September 15, 2015.

### FIELD

The present disclosure relates to medical devices and more specifically to electrohydraulic lithotripsy probes.

### BACKGROUND

The statements in this section merely provide background information related to the present disclosure and may not constitute prior art.

Electrohydraulic lithotripsy is a procedure used as a means to break up stones within the biliary tree and urinary tract. While many stones may naturally pass through and out of the patient, some stones are too large to be passed on their own. These stones may become stuck in the biliary tree or urinary tract, thereby requiring medical intervention. A common way to remove stones is with lithotripsy: breaking the stones up into smaller pieces that are then able to be passed naturally out of the patient's body. One specific example of lithotripsy is electrohydraulic lithotripsy, which employs high energy shock waves to fragment the stones. These shock waves can be generated and targeted at the stone from outside of the patient's body or with a device that is inserted into the patient's body- either percutaneously or through a natural body cavity.

Electrohydraulic lithotripsy uses a shock wave generating device that is inserted into the patient's body. The device, or probe, is most commonly passed through an accessory channel of a scope or other similar introducer device until the probe is adjacent to the stone. A shock wave is then generated at the tip of the probe towards the stone. Eventually, the stone will fragment and the probe and scope may then be removed while the stone fragments naturally pass through and out of the patient's body. Alternatively, the fragments may be removed by a vacuum, basket, or other fragment collection device inserted through or with the scope.

The scope, which is often a cholangioscope, must have an outer diameter small enough to allow it to be safely advanced through a body lumen of a patient. Sometimes, the cholangioscope is advanced through a working channel of a larger duodenoscope that also must have a diameter small enough to allow it be safely advanced through a body lumen of a patient. Since the probe is passed through a working channel of one of these scopes, the outer diameter of the probe must be fairly small. However, these probes are generally quite long, with lengths often exceeding 230 centimeters. Because of the high length to diameter ratio, one common problem associated with electrohydraulic lithotripsy is the buckling or kinking of the probe as it is advanced through the working channel of the scope and into a patient's body lumen. Kinking and buckling of the probe can be caused by the friction generated between the probe and the working channel of the scope or various structures in the patient's body lumen. As the physician advances the probe further into the scope, the friction between the scope and probe increases, thus requiring a greater force to further advance the probe. However, as the physician applies more force to the proximal end of the probe, the probe is more likely to kink or buckle, as it cannot withstand a large force due to its small diameter and low strength (or stiffness). When the probe kinks or buckles, the physician may have increased difficulty in advancing the probe towards the stone. The probe may also buckle or kink within the scope as the scope navigates the twists and turns of the patient's body lumen or at the distal end of the probe as it is advanced past the distal end of the scope.

Thus, it is desirable to provide a lithotripsy probe that is resistant to kinking and buckling while maintaining a small outer diameter that may be passed through the working channel of a scope.

### SUMMARY

In one form of the present disclosure, a medical device is provided. The medical device comprises a probe comprising an elongated shaft comprising a distal end, a proximal end, and an external wall. The probe further comprises at least two conductors extending substantially along a longitudinal length of the probe. The probe also comprises a strengthening element attached to an outer surface of the external wall of the elongated shaft. The probe is configured to slidably move within a working channel of a delivery device. Also, the strengthening element is configured to limit buckling of the probe as a distal portion of the probe is advanced past a deflection point of the delivery device.

In another form of the present disclosure, a lithotripsy kit is provided. The lithotripsy kit comprises a cholangioscope with a working channel. The lithotripsy kit further comprises a lithotripsy probe comprising an elongated shaft with a distal end, a proximal end, and an external wall. The lithotripsy probe further comprises at least two conductors extending substantially along a longitudinal length of the probe and a strengthening element attached to an outer surface of the external wall of the elongated shaft. Additionally, the lithotripsy probe is slidably movable within the working channel of the cholangioscope. Also, the strengthening element is configured to limit buckling of the probe as a distal portion of the probe is advanced past a deflection point of the cholangioscope.

In yet another embodiment of the disclosure, a method of modifying a lithotripsy probe is provided. The method comprises providing a lithotripsy probe comprising an elongated shaft. The elongated shaft comprises a distal end, a proximal end, and an external wall. The lithotripsy probe also comprises at least two conductors extending substantially along a longitudinal length of the probe. The method further comprises securing a strengthening element to an outer surface of an external wall of the elongated shaft. The lithotripsy probe is configured to slidably move within a working channel of a delivery device. Further, the strengthening element is configured to limit buckling of the lithotripsy probe as distal portion of the lithotripsy probe is advanced distally past a deflection point of the working channel of the delivery device.

The strengthening element may be formed from a material which is different from or stiffer than the material from which the elongated shaft is formed. The strengthening element may be formed of a metal or metal alloy. Further areas of applicability will become apparent from the description provided herein. It should be understood that the description and specific examples are intended for purposes of illustration only and are not intended to limit the scope of the present disclosure.

### DRAWINGS

The drawings described herein are for illustration purposes only and are not intended to limit the scope of the present disclosure in any way.
FIG. 1 is a drawing of a lithotripsy probe, duodenoscope and cholangioscope inserted into a patient's duodenum in accordance with the teachings of the present disclosure;
FIG. 2 is a drawing of a lithotripsy probe with strengthening elements, duodenoscope, and cholangioscope inserted into a patient's duodenum;
FIG. 3 is another drawing of a lithotripsy probe with strengthening elements and a duodenoscope inserted into a patient's duodenum;
FIG. 4A is an embodiment of the present invention with a single strengthening element placed on the proximal end of a probe;
FIG. 4B is an embodiment of the present invention with a single strengthening element placed along the entire length of a probe;
FIG. 4C is an embodiment of the present invention with a single strengthening element placed along the distal end of a probe;
FIG. 4D is an embodiment of the present invention with a single strengthening element placed along a central portion of a probe;
FIG. 4E is an embodiment of the present invention with two strengthening elements placed along a probe;
FIG. 5 is a cross-sectional view of a probe with four tubular strengthening elements;
FIG. 6 is a cross-sectional view of a probe with two tubular strengthening elements;
FIG. 7 is a cross-sectional view of a probe with a strengthening element wrapped around the entire circumference of the probe;
FIG. 8 is a cross-sectional view of a probe with a strengthening element wrapped around three quarters of the circumference of the probe;
FIG. 9 is a cross-sectional view of a probe with a strengthening element wrapped around half of the circumference of the probe;
FIG. 10 is a cross-sectional view of a probe with a strengthening element wrapped around one quarter of the circumference of the probe;
FIG. 11 is a cross-sectional view of a probe with a strengthening element with a varying radial thickness;
FIG. 12 is a cross-sectional view of a probe with one strengthening element;
FIG. 13 is a cross-sectional view of a probe with two strengthening elements;
FIG. 14 is a cross-sectional view of a probe with two strengthening elements;
FIG. 15 is a cross-sectional view of a probe with four strengthening elements;
FIG. 16 is a cross-sectional view of a probe with eight strengthening elements;
FIG. 17 is a cross-sectional view of a probe with a strengthening element with a varying thickness;
FIG. 18 is a cross-sectional view of a probe with a strengthening element with tapered ends;
FIG. 19 is a cross-sectional view of a probe with two strengthening elements, each with tapered ends;
FIG. 20 is a side view of a probe with a strengthening element with tapered ends;
FIG. 21 is a side view of a probe with a strengthening element with a gradual taper from the proximal end to the distal end;
FIG. 22 is a side view of a probe with a strengthening element with a varying thickness along the longitudinal length of the probe;
FIG. 23 is an orthographic view of a probe with a curved, rectangular strengthening element;
FIG. 24 is an orthographic view of a probe with a strengthening element extending around the entire circumference of the probe;
FIG. 25 is an orthographic view of a probe with a spiral strengthening element;
FIG. 26 is an orthographic view of a probe with multiple strengthening elements arranged in a pattern along the length of the probe;
FIG 27 is an orthographic view of a probe with two circumferential strengthening elements;
FIG. 28 is an orthographic view of a probe with two strengthening elements wrapped circumferentially around the probe; and
FIG. 29 is an orthographic view of a probe with two spiral strengthening elements.

### DETAILED DESCRIPTION

The following description is merely exemplary in nature and is not intended to limit the present disclosure, application, or uses. It should be understood that throughout the drawings, corresponding reference numerals indicate like or corresponding parts and features. It should also be understood that various cross-hatching patterns used in the drawings are not intended to limit the specific materials that may be employed with the present disclosure. The cross-hatching patterns are merely exemplary of preferable materials or are used to distinguish between adjacent or mating components illustrated within the drawings for purposes of clarity.

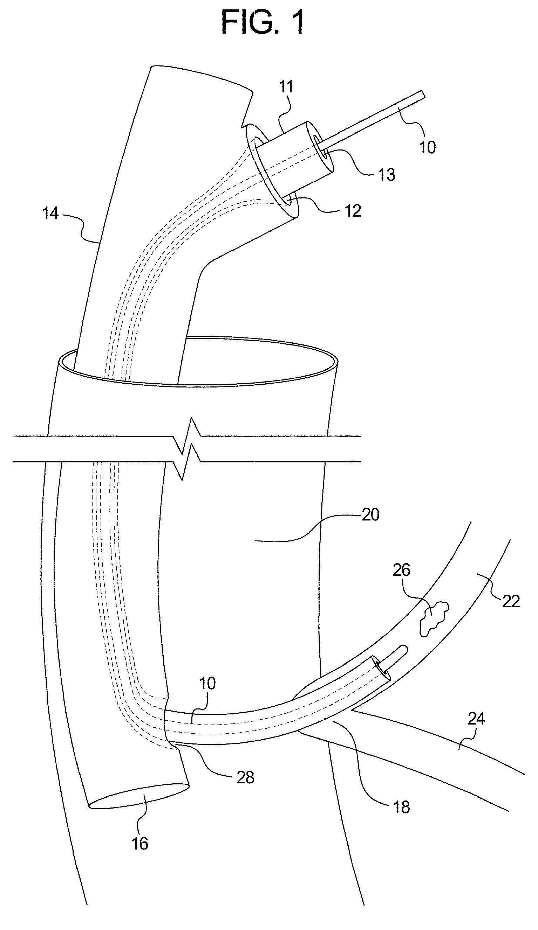

FIG. 1 shows a lithotripsy probe 10 inserted into a working channel 13 of a cholangioscope 11, which is in turn inserted into a working channel 12 of a duodenoscope 14. In this example, the duodenoscope 14 is inserted into the mouth of a patient and through the digestive track until the distal end 16 of the duodenoscope 14 is near the papilla of Vater 18 in the duodenum 20. The papilla of Vater 18 is a mound-like structure that extends into the duodenum 20 and serves as the exit point for the common bile duct 22 and pancreatic duct 24. A stone 26 may be lodged in the common bile duct 22, and thus the probe 10 must be inserted through the papilla of Vater 18 until the distal end of the probe 10 is near the stone 26. Before the probe 10 is inserted, the cholangioscope 11 may be inserted through the working channel 12 of the duodenoscope 14 and then pushed through the papilla of Vater 18 until the distal end of the cholangioscope 11 is adjacent to the stone 26. The probe 10 may then be inserted through a working channel 13 of the cholangioscope 11 until the distal end of the probe 10 is near the stone 26. Alternatively, the probe 10 can be at least partially preloaded into the working channel 13 of the cholangioscope 11 and the probe 10 and cholangioscope 11 can be advanced through the working channel 12 of the duodenoscope 14 together. Once the distal end of the probe 10 is near the stone 26, the shock wave energy is applied at the distal tip of the probe 10 and towards the stone 26 which causes the stone 26 to fragment. The probe 10, cholangioscope 11, and duodenoscope 14 may then be withdrawn from the patient's body.

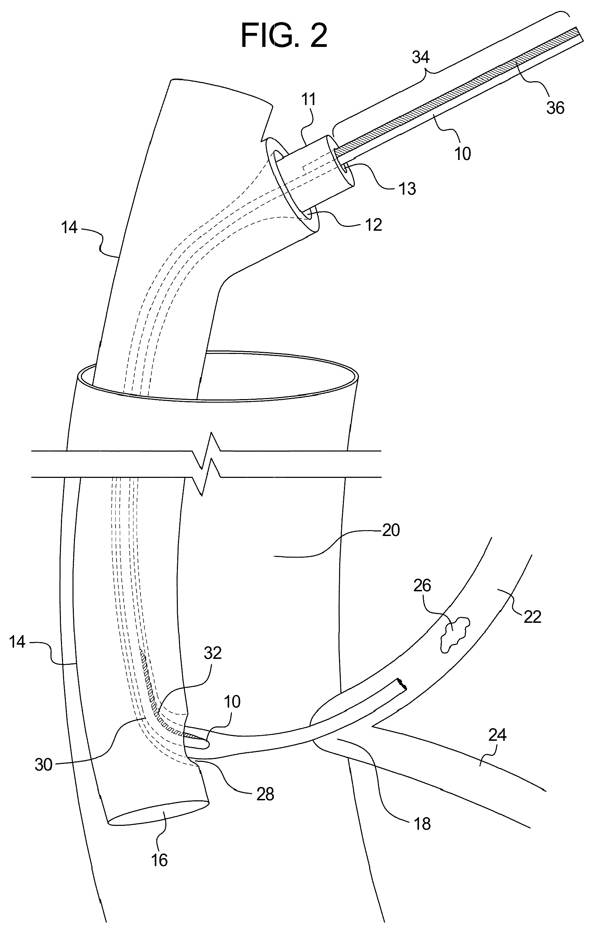

While the probe 10 is at risk of kinking or buckling throughout this procedure, there are several points when the probe 10 is at a significant risk. For example, the probe 10 is at a significant risk of buckling when the distal end of the probe 10 extends past the side port 28 of the duodenoscope 14 (FIG. 2). The cholangioscope 11, and therefore the probe 10, are deflected near the side port 28 by an elevator (not shown) within the duodenoscope 14. The elevator can be manipulated by the physician to control the deflection of the cholangioscope 11 and probe 10 and thus steer the cholangioscope 11 and probe 10 towards the papilla of Vater 18 or other body structure. The elevator may deflect the cholangioscope 11 and probe 10 as much as or more than 90 degrees and through a relatively tight bending radius, which creates a high risk of kinking at the deflection point 30. Thus, it may be advantageous to attach a distal stiffening (or strengthening) element 32 to the probe 10 near the distal end of the probe 10. As the probe 10 continues to be advanced towards the stone 26, a certain length of the probe 10 passes through the deflection point 30. Therefore, at various stages of the procedure, this certain length of the probe 10 may be at risk of kinking. Thus, it may be ideal for the distal stiffening element 32 to span from the distal end of the probe 10 along and past the portion of the probe 10 that may pass through the deflection point 30. The distal stiffening element 32 provides added strength to the probe 10 to prevent the probe 10 from buckling or kinking at a point where the probe 10 is susceptible to it.

Still referring to FIG. 2, the probe 10 is also at risk of buckling near the proximal end of the probe 10 as the probe 10 advances past the deflection point 30. When the distal end of the probe 10 reaches the elevator, the force necessary to further advance the probe 10 through the cholangioscope 11 increases due to the high amount of friction between the working channel 13 of the cholangioscope 11 and the probe 10 at the deflection point 30 and along the entire length of the working channel 13. Thus, the physician must apply a larger force to the proximal end of the probe 10 than was previously necessary. The portion of the probe 10 that is within the working channel 13 is supported by the low clearance between the walls of the working channel 13 and the probe 10 and thus is unlikely to kink or buckle. However, the proximal portion 34 of the probe 10 that has not yet been advanced into the working channel 13 of the cholangioscope 11 does not have this support to prevent it from buckling. Thus, when the physician applies an increased force to the proximal portion 34 of the probe 10 to advance the probe 10 past the elevator and deflection point 30, the proximal portion 34 is prone to buckling. Therefore, it may be ideal to attach a proximal stiffening element 36 to the proximal portion 34 of the probe 10. The proximal stiffening element 36 provides extra support to the proximal portion 34 of the probe 10 to prevent or limit buckling when the physician applies an increased force to advance the probe 10 past the elevator and deflection point 30. The proximal stiffening element 36 ideally extends along the probe 10 from a point within the working channel 13 of the cholangioscope when the tip of the probe is adjacent to the elevator proximally to at least a point on the probe 10 external the working channel 13 when the distal end of the probe 10 is adjacent to the stone 26.

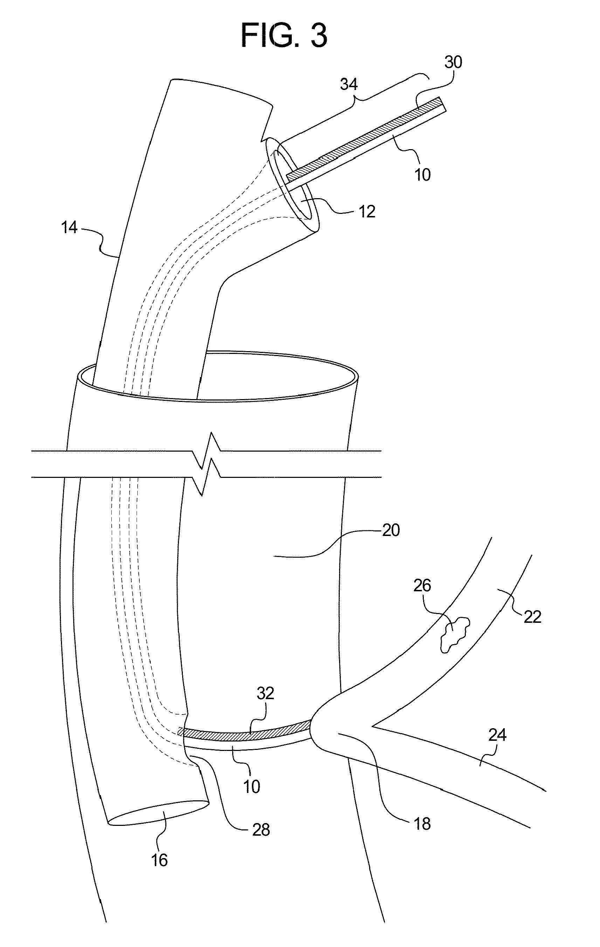

While the probe 10 is most frequently used in conjunction with a cholangioscope 11 as shown in FIGS. 1 and 2, the probe 10 may also be used without a cholangioscope 11. As shown in FIG. 3, the probe 10 may be advanced directly through the working channel 12 of the duodenoscope 14 and eventually through the papilla of Vater 18. In this example, the probe 10 is at a great risk of buckling when the probe 10 is being pushed through the papilla of Vater 18. Within the papilla of Vater 18 is a sphincter, the sphincter of Oddi (not shown) - a strong muscular valve that is designed to only permit fluid and substances from exiting rather than entering the common bile duct 22 and pancreatic duct 24. Thus, a large force is required to push the probe 10 through the sphincter of Oddi and into the common bile duct 22. The physician applies this requisite force to the probe 10 at the proximal portion 34 of the probe 10 that is still located external the scope 14. However, similar to when the probe 10 is advanced past the elevator, the requisite force is great enough that the proximal portion 34 of the probe 10 may buckle. Thus, the proximal strengthening element 36 may provide added strength to the proximal portion 34 of the probe 10 to prevent it from buckling when the probe 10 is advanced past the sphincter of Oddi. Additionally, the distal strengthening element 32 may extend to the distal tip of the probe 10 to prevent the distal portion of the probe 10 from buckling when the physician is attempting to access the common bile duct 22 through the sphincter of Oddi.

As described above, whether the lithotripsy probe 10 is advanced directly through the working channel 12 of the duodenoscope 14 or through the working channel 13 of the cholangioscope 11, the probe 10 is at a greater risk of buckling and kinking at discrete stages. When the cholangioscope 11 is used, the probe 10 is at a greater chance of buckling as it is advanced past the elevator. When the cholangioscope 11 is not used, the probe 10 is at a greater chance of buckling as it is advanced past the elevator and as the distal end of the probe 10 is advanced through the sphincter of Oddi. While the strengthening elements 32, 36 may be designed to prevent kinking and buckling at both proximal and distal locations for both of these discrete stages, it may be advantageous or desirable to only prevent kinking for one of the stages. For example, the distal strengthening element 32 may be designed to extend from the distal tip of the probe 10 to a point on the probe 10 that is within the working channel 12 when the distal tip of the probe 10 is in contact with the sphincter of Oddi. The probe 10 is prone to buckling at the portion distal the exit of the working channel since the probe 10 not supported by the working channel 12 as it exits the duodenoscope 14. Thus, the distal strengthening element 32 may help prevent buckling at this location as the probe 10 is advanced past the sphincter of Oddi. However, in this example the distal strengthening element 32 does not extend along the entire portion of the probe 10 that may be advanced past the elevator and deflection point 30. Thus, while the location of the distal strengthening element 32 may limit or prevent buckling when the probe 10 is passed through the sphincter of Oddi, the probe 10 may still be prone to kinking near the deflection point 30.

Alternatively, the distal strengthening element 32 may be omitted entirely. The outer diameter of the probe 10, especially the distal portion, is preferably minimized to allow the probe 10 to be advanced through the narrow working channel 12 of the duodenoscope or the working channel 13 of the cholangioscope 11. Additionally, when the cholangioscope 11 is not used, the physician may find it easier to advance the probe 10 through the sphincter of Oddi without the distal strengthening element 32 since the probe 10 has a small outer diameter. Thus, adding a strengthening element to the distal portion of the probe 10 may be undesirable. However, the proximal portion 34 of the probe 10 may only be partially advanced into the working channel 12 of the duodenoscope 14 or the working channel 13 of the cholangioscope 11 and therefore may not have the same outer diameter restrictions of the rest of the probe 10. Thus, the proximal strengthening element 36 may be used with less concern for the increased outer diameter the proximal strengthening element 36 may cause. Alternatively, the distal strengthening element 32 may be included and the proximal strengthening element 36 may be omitted entirely.

The probe and strengthening elements may be used in a variety of applications with varying lengths and designs. In one example, the probe 10 as shown in FIGS. 1-3 may be around 200 to 300 centimeters in length. The length of the working channel 12 of the duodenoscope 14 may be about 140 to 160 centimeters. The length of the working channel 13 of the cholangioscope 11 may be around 200 to 250 centimeters. This distance between the distal side port 28 and the papilla of Vater 18 is generally around .5 to 5 centimeters. Additionally, the probe 10 may extend up to 30 centimeters past the papilla of Vater 18 and into the common bile duct 22. Therefore, when the distal end of the probe 10 is advanced through the working channel 12 and reaches the deflection point, about 20 to 60 centimeters of the probe 10 may extend outside of the proximal end of the working channel 13 of the cholangioscope 11. Therefore, it may be ideal to make the proximal strengthening element at least 20 to 60 centimeters in length, thus ensuring that the proximal portion of the probe 10 does not kink or buckle. Additionally, since the distance between the papilla of Vater 18 and the distal side port 28 may be around .5 to 5 centimeters, it may be ideal to make the distal strengthening element 32 20 to 40 centimeters in length.

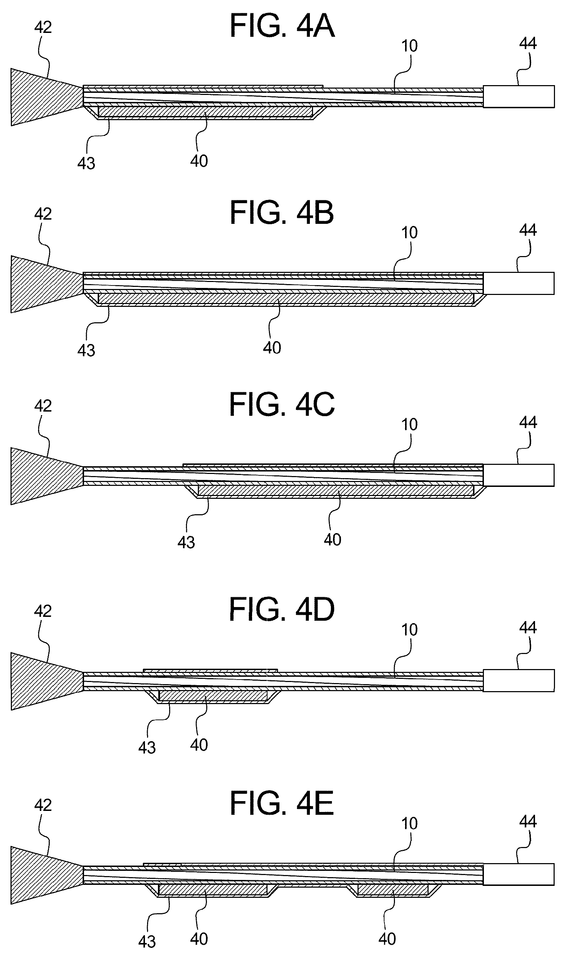

FIGS. 4A through 4E show several exemplary placements of a strengthening element 40 on the probe 10. The probe 10 has a proximal end 42 and a distal tip 44. In FIG. 4A, a single strengthening element 40 extends from the proximal end 42 distally along a portion of the length of the probe 10. In FIG. 4B, a single strengthening element 40 extends along the entire length of the probe 10. In FIG. 4C, a single strengthening element 40 extends from the distal tip 44 proximally along a portion of the length of the probe 10. In FIG. 4D, a single strengthening element 40 extends along a central portion of the probe 10. In FIG. 4E, two strengthening elements 40 are attached to the probe 10. Because the outer diameter of the probe 10 is increased with the addition of the strengthening element 40, it may be advantageous to add the strengthening element 40 to only a limited length of the probe 10. These are just five examples of the potential placement of the strengthening element 40 along the probe 10. Any number of locations are contemplated, including the use of multiple strengthening elements.

The strengthening elements are ideally placed on the outer surface of the external wall of the probe. While the strengthening elements may also be placed within the outer wall of the probe, there may be several disadvantages. The probe is frequently designed with conductive wires that run along the length of the probe to the distal end. These conductive wires carry the electrical energy that results in a high voltage spark and the accompanying shockwave at the distal end of the probe. For the probe to operate effectively, the conductive wires must be properly separated and insulated along the entire length of the probe, thereby only allowing electrical contact between the conductive wires at the distal end of the probe. Since the strengthening elements are frequently made of a conductive material, there is a risk that the strengthening elements will interfere with the conductive wires. For example, the strengthening elements may inadvertently contact one or both of the conductive wires, causing the conductive wires to short circuit, thus rendering the probe ineffective. Additional interference issues may occur even if the conductive wires remain properly insulated from the strengthening elements. If the strengthening elements are within the outer wall of the probe, they are closer to the conductive wires and therefore are at a greater risk of causing interference or short circuiting the conductive wires. Placing the strengthening elements on the outer surface of the probe's external wall reduces or eliminates this concern.

The strengthening elements may be comprised of various biocompatible materials that are capable of adding strength and stiffness to the probe 10, including, but not limited to: peek beading, stainless steel, various metal alloys, and other stiff or flexible polymers. In some embodiments, as shown in FIGS. 4A through 4E, the strengthening element 40 may be made of nitinol. In this embodiment, the strengthening elements 40 are attached to an external wall of the probe 10; however, the strengthening elements 40 may also be placed within the external walls of the probe 10. An outer covering, such as shrink tubing 43, may be used to secure the strengthening element 40 to the probe 10. The outer covering may be comprised of PTFE shrink, PET shrink, or other similar materials. Alternatively, the strengthening element 40 may be attached to the probe 10 using casting, molding, adhesives, or similar methods.

In addition to the various potential locations of the strengthening elements along the length of the probe 10, various shapes and patterns may be used to minimize the profile of the strengthening elements or maximize their effectiveness. The following figures and descriptions are merely exemplary in nature and any variety of patterns or shapes are contemplated in this disclosure.

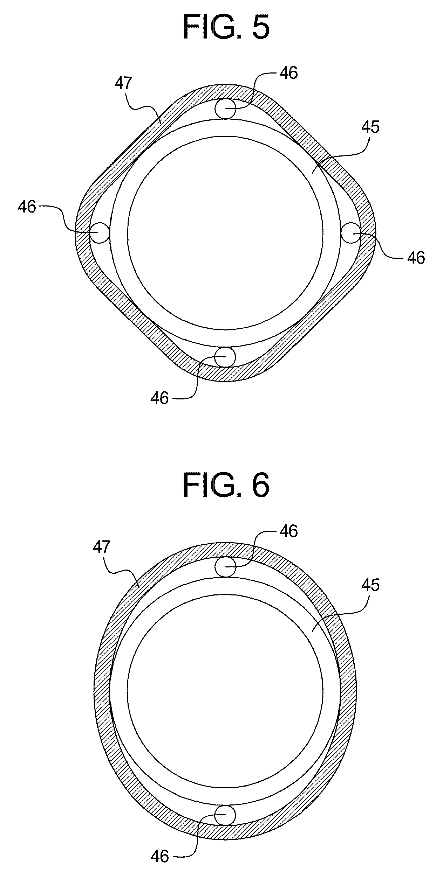

FIGS. 5 and 6 show two potential cross sections of the probe 45 and the strengthening elements 46. FIG. 5 shows the probe 45 with four strengthening elements 46 and FIG. 6 shows the probe 45 with two strengthening elements 46. In this example shrink wrap tubing 47 is wrapped around the strengthening elements 46 to secure the strengthening elements 46 to the probe 45, however various other methods may be used as discussed previously. Additionally, any number of strengthening elements 46 may be used and may be evenly spaced around the cross-section of the probe 45 or with other patterns. The strengthening elements 46 may have varying diameters to increase or decrease the amount of strength added to the probe 45 as desired. Further, the strengthening elements 46 may have various shapes, including tubular, cylindrical, rectangular, etc. The strengthening elements 46 may extend along the entire length of the probe 45 or along just a portion of it. When multiple strengthening elements 46 are used, the strengthening elements may span different lengths of the probe 45.

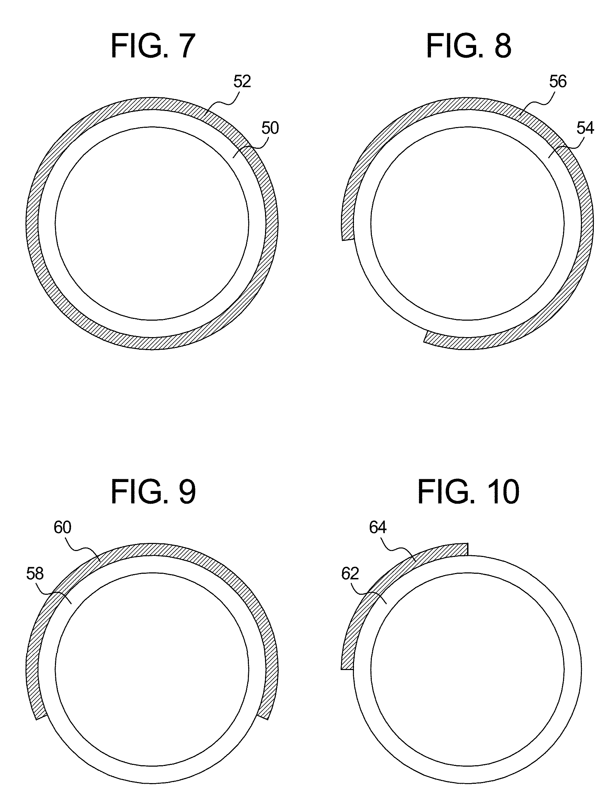

FIGS 7-10 show various strengthening elements with circular cross-sections. In these Figures, and the additional Figures following, the shrink wrap tubing or other bonding method is omitted to more clearly show the various potential designs of the strengthening elements. In FIG. 7, the strengthening element 52 extends around the entire circumference of the probe 50. In FIG. 8, the strengthening element 56 extends around three-quarters of the circumference of the probe 54. In FIG. 9, the strengthening element 60 extends around about half of the circumference of the probe 58. In FIG. 10, the strengthening element 64 extends around one-quarter of the circumference of the probe 62. These designs are merely examples, and the strengthening element may extend around any portion of the circumference. Additionally, the strengthening elements may extend along the entire length of the probe or along only a portion of the probe. Additionally, the strengthening elements may have varying radial thicknesses to achieve greater strength or a lower radial profile as desired.

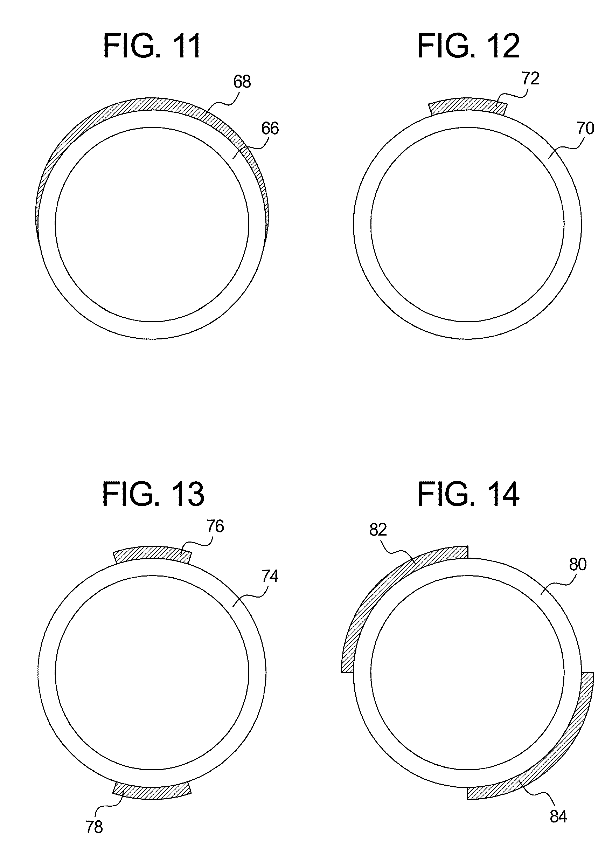

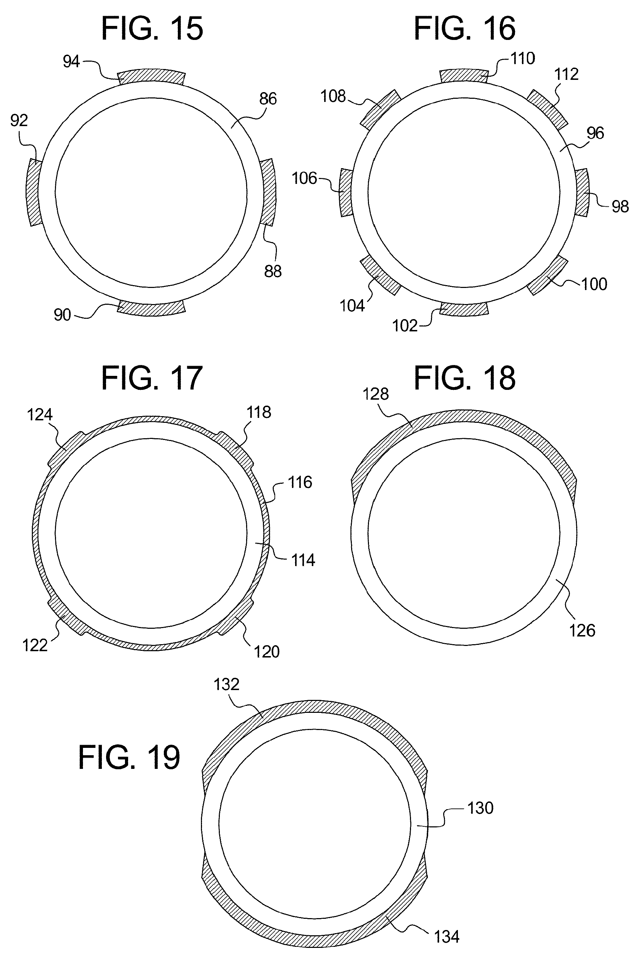

FIG. 11 shows one example of a varying diameter strengthening element 68. The mid-point of the strengthening element 68 has a large radial thickness which gradually thins towards each end of the strengthening element 68. FIG. 12 shows a narrow strengthening element 72 attached to the probe 70. FIG. 13 uses two narrow strengthening elements 76, 78 attached to the probe 74. FIG. 14 uses two wider strengthening elements 82, 84. Alternatively, 4 or more strengthening elements may be used of varying cross-sectional lengths. For example, FIG. 15 shows a probe 86 with four strengthening elements 88, 90, 92, 94. Also, FIG. 16 shows a probe 96 with eight strengthening elements 98, 100, 102, 104, 106, 108, 110, 112. Any number of additional or fewer strengthening elements can be added to the design as desired, including spacing the strengthening elements in a non-symmetrical fashion.

FIG. 17 shows a single strengthening element 116 that wraps around the entire circumference of the probe 114. However, the strengthening element 116 includes a varying outer diameter, such that several bumps 118, 120, 122, and 124 with larger outer diameters than the rest of the strengthening element 116 are formed. Various other patterns using a varying outer diameter of the strengthening element 116 may be employed. FIGS. 18 and 19 show strengthening elements with a taper at each edge. FIG. 18 has a single strengthening element 128 that tapers at the edge and FIG. 19 has two strengthening elements 132, 134 that taper at their respective edges.

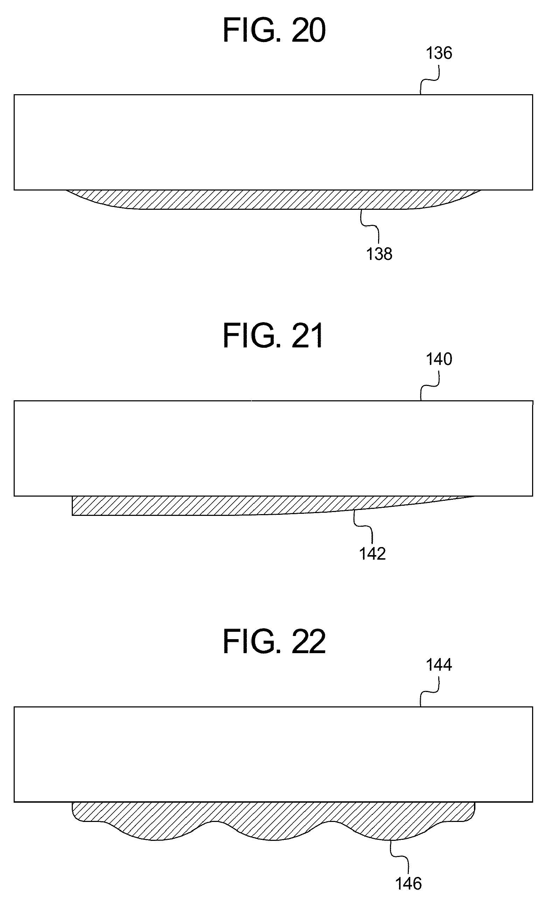

FIGS 20-22 show several exemplary side views of the probe and strengthening element. FIG. 20 shows the strengthening element 138 with a taper on each longitudinal end of the strengthening element 138. FIG. 21 shows a strengthening element 142 that tapers from one end of the probe 140 towards the other end. FIG. 22 shows a strengthening element 146 with a varying thickness along the longitudinal length of the strengthening element 146. These variations in the longitudinal lengths and diameters along the longitudinal lengths can be combined with any of the aforementioned cross-sectional designs.

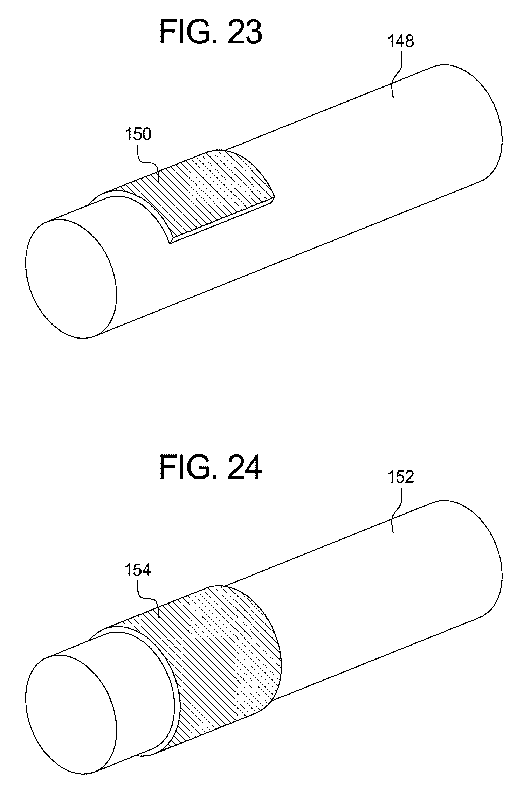

FIGS. 23-29 show orthographic views of exemplary strengthening element designs. FIG. 23 shows a curved strengthening element 150 that extends around a portion of the circumference of the probe 148 and along only a limited length of the probe 148. FIG. 24 shows a strengthening element 154 that extends around the entire circumference of the probe 152 and along a limited length of the probe 152. In either of these examples, the strengthening elements could be modified to extend along the entire length of the probe, or multiple strengthening elements of the same shape and length may be used along various portions of the probe.

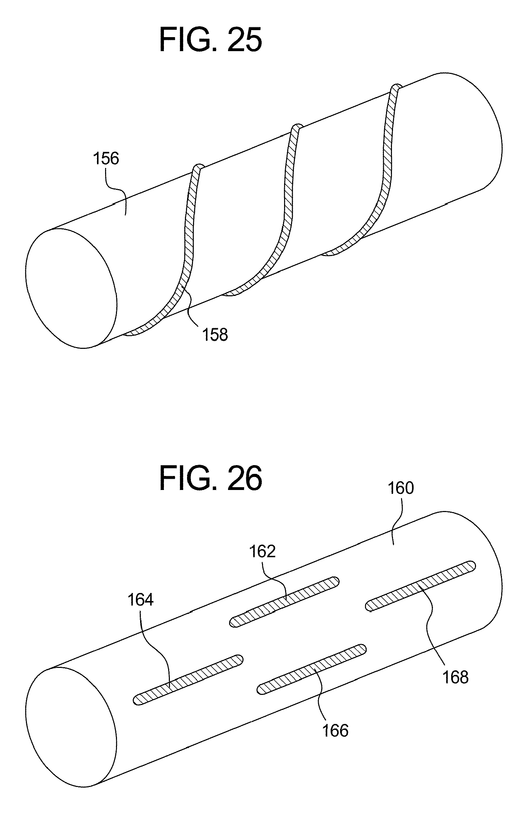

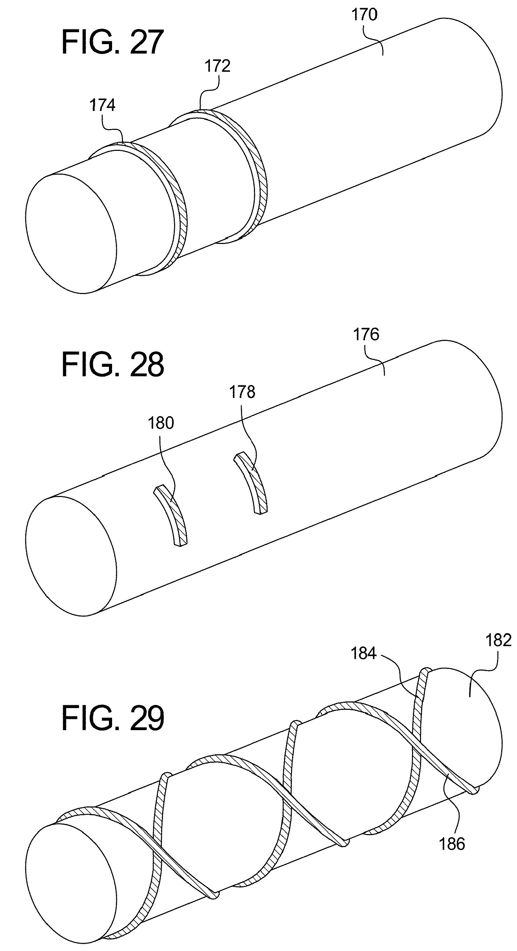

FIG. 25 shows a strengthening element 158 that is wrapped in a spiral pattern around the probe 156. The strengthening element 158 may have a cylindrical, rectangular, tubular, or other cross-section. Additionally, the strengthening element 158 may be wrapped in a tighter spiral to increase the effectiveness of the strengthening element 158 or in a looser spiral as desired. While the strengthening element 158 in this example extends along the entire length of the probe 156, the strengthening element 158 may be designed to only extend along a portion of the probe 156. Additionally, multiple spiral strengthening elements may be used, such as shown in FIG. 29. In FIG. 29, one strengthening element 184 is wrapped around the probe 182 in a clockwise direction while the other strengthening element 186 is wrapped around the probe 182 in a counter-clockwise direction. In this embodiment the two strengthening elements 184, 186 are wrapped around the probe 182 in spirals of similar tightness, but they may be varied as desired. Alternatively, two or more spiral strengthening elements may be wrapped around the probe in the same direction, with the tightness or looseness of the spirals varying or remaining constant.

FIG. 26 shows a probe 160 with multiple strengthening elements 162, 164, 166, 168. This embodiment shows one example of a pattern of multiple, straight strengthening elements arranged along the length of the probe 160. Various other placement patterns may be used as well, including non-uniform placement and shape of the various strengthening elements.

FIG. 27 shows a probe 170 with two strengthening elements 172, 174 wrapped circumferentially around the probe 170. More than two, or only one, strengthening elements may be used in the arrangement shown in FIG. 27. FIG. 28 shows a probe 176 with two strengthening elements 178, 180 wrapped around a portion of the circumference of the probe 176. More than two, or only one, strengthening elements may be used in the arrangement shown in FIG. 28.

While the present disclosure describes the invention in terms of lithotripsy probe used during a biliary procedure, the improved probe may be used in any lithotripsy procedure to prevent kinking and buckling of the probe when inserted into a patient. Further, the anti-kinking and buckling improvements may be used with a variety of other medical devices unrelated to lithotripsy, such as catheters used in a variety of medical procedures. Also, the improvements described above may be used in a variety of non-medical applications.

The description of the disclosure is merely exemplary in nature and, thus, variations that do not depart from the substance of the disclosure are intended to be within the scope of the disclosure. Such variations are not to be regarded as a departure from the scope of the disclosure.

## Claims

1. A medical device, comprising:
a probe comprising an elongated shaft comprising a distal end, a proximal end, and an external wall, the probe further comprising at least two conductors extending substantially along a longitudinal length of the elongated shaft, the probe further comprising a strengthening element attached to an outer surface of the external wall of the elongated shaft;
wherein the probe is configured to slidably move within a working channel of a delivery device;
wherein the strengthening element is configured to limit buckling of the probe as a distal portion of the probe is advanced past a deflection point of the delivery device.

2. The medical device of claim 1, wherein:
the strengthening element extends from the proximal end to the distal end of the elongated shaft.

3. The medical device of claim 1, wherein:
the strengthening element extends from the proximal end of the elongated shaft along only a portion of the longitudinal length of the shaft.

4. The medical device according to claims 1 through 3, wherein:
the strengthening element comprises a first strengthening element comprising a cylindrical shape and a second strengthening element comprising a cylindrical shape, wherein the first strengthening element and second strengthening element each extend along at least a portion of the longitudinal length of the elongated shaft.

5. The medical device according to claims 1 through 3, wherein:
the strengthening element comprises a cylindrical shape, wherein the strengthening element extends along at least a portion of the longitudinal length of the elongated shaft.

6. The medical device according to claims 1 through 5, further comprising:
a shrink wrap tubing surrounding the strengthening element and configured to secure the strengthening element to the elongated shaft.

7. The medical device according to claims 4 through 6, wherein:
the strengthening element extends circumferentially around a cross-section of the elongated shaft and along at least a portion of the longitudinal length of the elongated shaft.

8. The medical device according to claims 4 through 7, wherein:
the strengthening element extends circumferentially around the entire cross-section of the elongated shaft.

9. The medical device according to claims 4 through 7, wherein:
the strengthening element extends circumferentially around half of the cross-section of the elongated shaft.

10. The medical device according to claims 4 through 7, wherein:
the strengthening element extends circumferentially around a quarter of the cross-section of the elongated shaft.

11. The medical device of claim 1, wherein:
the strengthening element extends along a portion of the longitudinal length of the elongated shaft, wherein proximal and distal ends of the strengthening element each comprise tapers from a larger cross-section of the strengthening element to a smaller cross-section of the strengthening element.

12. The medical device of claim 1, wherein:
the strengthening element extends along a portion of the longitudinal length of the elongated shaft, wherein an end of the strengthening element comprises a taper from a larger cross-section of the strengthening element to a smaller cross-section of the strengthening element.

13. The medical device of claim 1, wherein:
the strengthening element extends helically around and along a portion of the longitudinal length of the elongated shaft.

14. The medical device of claim 1, wherein:
the strengthening element comprises a first strengthening element and a second strengthening element, where the first and second strengthening elements each extend helically around and along a portion of the longitudinal length of the elongated shaft, wherein the first and second strengthening element intersect each other at at least one location along the portion of the elongated shaft.

15. The medical device of any preceding claim in the form of an electrohydraulic lithotripsy probe.
